(19) Europäisches Patentamt
European Patent Office
Office européen des brevets

(11) Veröffentlichungsnummer: **0 076 915**
**A2**

(12) **EUROPÄISCHE PATENTANMELDUNG**

(21) Anmeldenummer: **82107834.2**

(22) Anmeldetag: **26.08.82**

(51) Int. Cl.[3]: **C 07 D 271/06**, C 07 D 271/10, C 07 D 413/12, A 01 N 43/82

(30) Priorität: **03.09.81 DE 3134882**

(43) Veröffentlichungstag der Anmeldung: **20.04.83 Patentblatt 83/16**

(84) Benannte Vertragsstaaten: **AT BE CH DE FR GB IT LI NL SE**

(71) Anmelder: **BASF Aktiengesellschaft, *Carl-Bosch-Strasse* 38, D-6700 Ludwigshafen (DE)**

(72) Erfinder: **Rentzea, Costin, Dr., Neuenheimer Landstrasse 72, D-6900 Heidelberg (DE)**
Erfinder: **Graf, Hermann, Dr., Ginsterstrasse 15, D-6704 Mutterstadt (DE)**
Erfinder: **Zeeh, Bernd, Dr., Thorwaldsenstrasse 5, D-6700 Ludwigshafen (DE)**
Erfinder: **Spiegler, Wolfgang, Dr., Amsterdamer Strasse 16, D-6700 Ludwigshafen (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

(54) **Oxadiazolcarbonsäureamide, Verfahren zu ihrer Herstellung und diese enthaltende Fungizide.**

(57) Oxadiazolcarbonsäureamide der allgemeinen Formel

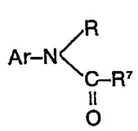
I,

worin

Ar 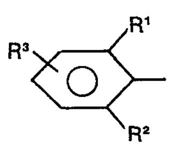 oder 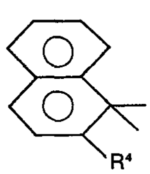 bedeutet, und

$R^1$ Alkyl, Alkoxy oder Halogen, $R^2$ Alkyl, Alkoxy, Halogen oder $NO_2$, $R^3$ Wasserstoff, Alkyl oder Alkoxy und $R^4$ Methyl bedeutet,

R 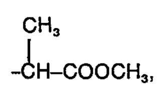, 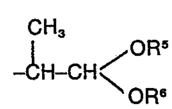

oder 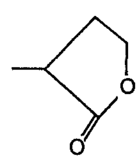 bedeutet, und

$R^5$ und $R^6$ Methyl oder Ethyl bedeuten oder $R^5$ und $R^6$ zusammen eine Alkylengruppe bedeuten, die zusammen mit dem Rest

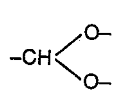 einen gegebenenfalls substituierten Dioxolan- oder 1,3-Dioxanring bilden und $R^7$ einen gegebenenfalls substituierten Oxadiazolrest bedeutet und diese enthaltende Fungizide.

EP 0 076 915 A2

Oxadiazolcarbonsäureamide, Verfahren zu ihrer Herstellung und diese enthaltende Fungizide

Die vorliegende Erfindung betrifft neue wertvolle Oxadiazolcarbonsäureamide, Verfahren zu ihrer Herstellung und Fungizide, die diese Verbindungen enthalten.

Heterocyclische Carbonsäureanilide, die fungizid wirksam sind, sind aus der DE-OS 25 13 732 bekannt. Heterocyclische Reste in diesen Verbindungen sind z.B. Pyridylreste. Die Wirkung dieser Verbindungen gegen Pilze aus der Klasse der Phycomyceten genügt bei niederen Aufwandkonzentrationen nicht den Anforderungen der Praxis.

Es wurde nun gefunden, daß neue Oxadiazolcarbonsäureamide der allgemeinen Formel

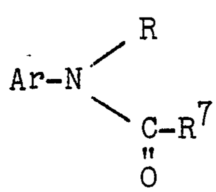 I,

worin

Ar 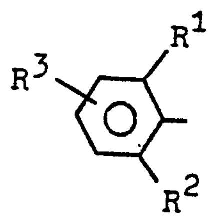 oder 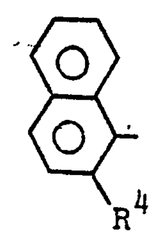 bedeutet, und

$R^1$ $C_1$-$C_3$-Alkyl, $C_1$-$C_3$-Alkoxy oder Halogen, $R^2$ $C_1$-$C_3$-Alkyl, $C_1$-$C_3$-Alkoxy, Halogen oder $NO_2$, $R^3$ Wasserstoff, $C_1$-$C_3$-Alkyl oder $C_1$-$C_3$-Alkoxy und $R^4$ Methyl bedeutet,

R $-\overset{CH_3}{\overset{|}{C}H}-COOCH_3$, 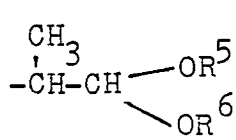 oder 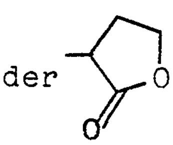 bedeutet,
und

Sws/P

$R^5$ und $R^6$ Methyl oder Ethyl bedeuten oder $R^5$ und $R^2$ zusammen eine Alkylengruppe darstellen, die zusammen mit dem Rest

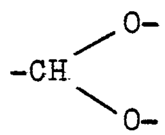 einen gegebenenfalls durch $C_1$-$C_3$-Alkyl

substituierten Dioxolan- oder 1,3-Dioxanring bilden und $R^7$ einen gegebenenfalls durch $C_1$-$C_3$-Alkyl substituierten 1,2,4-Oxadiazol-3-yl-, 1,2,4-Oxadiazol-5-yl- oder 1,3,4-Oxadiazol-2-yl-rest bedeutet, starke fungizide Eigenschaften aufweisen.

In der Formel I bedeuten $R^1$ und $R^2$ bevorzugt Methyl, Ethyl, Methoxy, Ethoxy und Chlor und $R^3$ bevorzugt Wasserstoff oder Methyl.

Die neuen Oxadiazolcarbonsäureamide der Formel I besitzen ein chirales Kohlenstoffatom in dem Rest R und je nach der Beschaffenheit von R noch weitere Chiralitätszentren. Nach üblichen Methoden können die optisch reinen Enantiomeren bzw. die Diastereomeren erhalten werden. Die vorliegende Erfindung erfaßt auch diese Verbindungen in reiner Form oder als Mischungen. Als Fungizide sind sowohl die reinen Enantiomeren bzw. die einheitlichen Diastereomeren als auch die bei der Synthese üblicherweise anfallenden Mischungen wirksam.

Weiterhin wurde gefunden, daß man die neuen Verbindungen der allgemeinen Formel (I) erhält, wenn man ein aromatisches Amin der Formel II

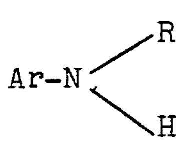 II,

worin
Ar und R die oben angegebenen Bedeutungen haben,

a) mit einem Säurehalogenid der Formel III

$$R^7-\overset{O}{\overset{\|}{C}}-Hal \qquad III$$

oder

b) mit einem Säureanhydrid der Formel IV

$$(R^7-CO)_2O \qquad IV,$$

worin
$R^7$ die oben angegebene Bedeutung hat und Hal, Chlor oder Brom bedeutet,

gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers, bei Temperaturen zwischen -10 und 100°C umsetzt. Als bevorzugte, gegenüber den Reaktionsteilnehmern inerte Lösungs- oder Verdünnungsmittel werden z.B. aliphatische oder aromatische Kohlenwasserstoffe wie Pentan, Cyclohexan, Petrolether, Benzol, Toluol oder Xylole; Halogenkohlenwasserstoffe, beispielsweise Methylenchlorid, Chloroform, 1,2-Dichlorethan, Chlorbenzol; Ketone wie Aceton und Methylethylketon; Ether wie Diethylether, Dimethoxyethan, Tetrahydrofuran oder Dioxan; Ester wie Essigsäureethylester; Nitrile wie Acetonitril; Sulfoxide wie Dimethylsulfoxid oder entsprechende Gemische verwendet.

Geeignete anorganische oder organische Basen, die gegebenenfalls auch als säurebindende Mittel bei der Reaktion verwendet werden können, sind beispielsweise Alkali- und Erdalkalicarbonate wie Natrium- oder Kaliumbicarbonat, Natrium- oder Kaliumcarbonat, Calciumcarbonat; Borate wie Natriumborat; Phosphate wie Natrium- oder Kaliumdi- oder -triphosphat oder Amine wie Triethylamin, N,N-Dimethylanilin, N,N-dimethylcyclohexylamin, N-Methylpiperidin oder Pyridin. Es können aber auch andere übliche Basen verwendet werden.

Das Herstellungsverfahren a) kann auch ohne säurebindende Mittel durchgeführt werden, wobei in einigen Fällen das Durchleiten von trockenem Stickstoff zur Vertreibung des gebildeten Halogenwasserstoffs angezeigt ist.

Als Reaktionsbeschleuniger kommen vorzugsweise Metallhalogenide wie Natriumbromid oder Kaliumjodid, Azole wie Imidazol oder 1,2,4-Triazol, Pyridine wie 4-Dimethylaminopyridin oder Dimethylformamid in Frage.

Die erfindungsgemäßen Umsetzungen erfolgen beispielsweise bei Temperaturen zwischen -10 und +100°C, vorzugsweise zwischen 0 und +40°C, drucklos oder unter Druck, kontinuierlich oder diskontinuierlich.

Eine weitere Untergruppe der neuen Verbindungen erhält man, indem man Verbindungen der Formel I, worin $R^5$ und $R^6$ Methyl oder Ethyl bedeuten mit einem 1,2- bzw. 1,3-Diol unter Abspaltung von Methanol oder Ethanol, gegebenenfalls in Gegenwart eines Lösungsmittels und gegebenenfalls unter Zusatz eines sauren Katalysators zu einer Verbindung der Formel I umsetzt, in der nun $R^5$ und $R^6$ zusammen mit

der Gruppe 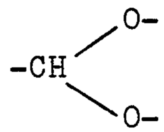 einen Dioxolan- bzw. einen 1,3--Dioxanring bilden.

Zu den oben genannten Diolen gehören beispielsweise Ethylenglykol, 1,2-Propylenglykol, 1,3-Propandiol, 2,3-Butandiol und Neopentylglykol. Die Abspaltung von $R^5OH$ und $R^6OH$ wird beispielsweise vorgenommen, indem man katalytische Mengen von starken protischen oder Lewis-Säuren wie Chlorwasserstoff (bzw. Salzsäure), Bromwasserstoff, p-Toluolsulfonsäure, Trifluoressigsäure, Trifluormethylsulfonsäure, Zinkchlorid, Zinkbromid oder Bortrifluorid--etherat bei Temperaturen von $0^oC$ bis zum Siedepunkt der abgespalteten Alkohole $R^3$-OH und $R^4$-OH auf das Acetal mit $R^5$ und $R^6$ Methyl oder Ethyl einwirken läßt. Die Umsetzung mit einem 1,2- oder 1,3-Diol wird beispielsweise vorgenommen, indem man die Alkohole $R^3$-OH und $R^4$-OH aus dem Reaktionsgemisch durch Destillation im Vakuum oder bei atmosphärischem Druck oder mit Hilfe eines inerten Gases, wie Stickstoff oder Argon, entfernt. Als Lösungs- oder Verdünnungsmittel eignen sich die oben genannten Flüssigkeiten oder Wasser, oder die 1,2- oder 1,3-Diole selbst.

Als saure Katalysatoren eignen sich Lewis- oder Protonensäuren, beispielsweise $BF_3$, $AlCl_3$ oder $ZnCl_2$ oder Mineralsäuren oder Sulfonsäuren.

Die Ausgangsstoffe der allgemeinen Formel II sind teilweise bekannt und werden nach an sich allgemein bekannten Methoden hergestellt.

Die Herstellung der neuen Verbindungen wird durch folgende Beispiele erläutert:

Beispiel 1

Herstellung von

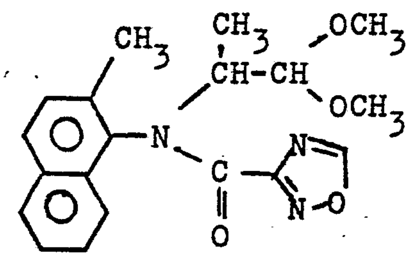

2-[N-(2-Methyl-1-naphthyl)-N-(1,2,4-oxadiazol-3-yl-carbonyl)]-aminopropanal-dimethylacetal (Verbindung Nr. 1)

49,2 g (0,19 Mol) 2-(N-2-Methyl-1-naphthyl)-aminopropanaldimethylacetal werden in 190 ml trockenem Toluol gelöst und gleichzeitig (aus zwei Tropftrichtern) mit der Lösung von 25,2 g (0,19 Mol) 1,2,4-Oxadiazol-3-carbonylchlorid (DE-PS 11 24 502) in 30 ml Toluol und mit der Lösung von 20 g (0,2 Mol) Triethylamin in 30 ml Toluol tropfenweise unter Rühren bei 25°C versetzt. Nach zweistündigem Nachrühren bei 25°C wird der Niederschlag abgesaugt und das Filtrat dreimal mit je 100 ml Wasser gewaschen, über $Na_2SO_4$ getrocknet, mit Tierkohle entfärbt und im Vakuum eingeeengt. Der harzige Rückstand wird viermal mit je 100 ml warmem (45°C) Petrolether durchgerührt, der Petrolether abdekantiert und das Harz bei 70°C und 0,3 mbar 4 Stunden getrocknet. Man erhält die Verbindung Nr. 1 als gelbliches Harz.

Ausbeute: 41,8 g (62 % d.Th.).

Beispiel 2

Herstellung von

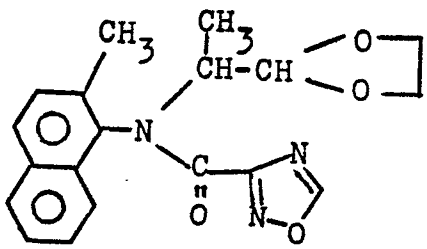

1,2,4-Oxadiazol-3-carbonsäure-N-[1-(1,3-dioxolan-2-yl)-2--methyl-ethyl]-N-2'-methyl-1'-naphthylamid (Verbindung Nr. 2)

24,8 g (0,07 Mol) der gemäß Beispiel 1 hergestellten Verbindung Nr. 1, 4,9 g Ethylenglykol und 0,2 g p-Toluolsulfonsäure in 150 ml trockenem Toluol werden bei 80°C 8 Stunden gerührt. Dabei wird das entstehende Methanol mit einem schwachen Stickstoff-Strom ständig aus dem Reaktionsgemisch vertrieben. Nach Abkühlen und dreimaligem Waschen mit je 100 ml Wasser wird die organische Lösung mit Kohle entfärbt und im Vakuum bei 80°C und 0,5 mbar eingeengt.

Man erhält 20 g (81 % d.Th.) der Verbindung Nr. 2 als Harz.

IR-Spektrum (Film): 3110, 3050, 2990, 2950, 1660, 1475, 1345, 1228, 1100, 860, 840, 815, 782, 745 $cm^{-1}$.

Beispiel 3

Herstellung von

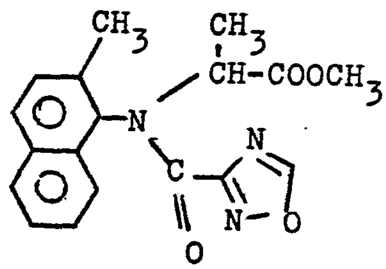

N-(1,2,4-Oxadiazol-3-yl-carbonyl)-N-(2-methyl-1-naphthyl)-alanin-methylester (Verbindung Nr. 3)

Einer Lösung von 22,9 g (0,045 Mol) N-(2-Methyl-1-naphthyl)-alanin-methylester in 100 ml trockenem Toluol werden 16,4 g (0,123 Mol) 1,2,4-Oxadiazol-3-carbonylchlorid gelöst in 30 ml Toluol zwischen +15°C und +30°C zugetropft. Das Gemisch wird bei 80°C 8 Stunden nachgerührt. Dabei wird das entstehende Chlorwasserstoff mit einem schwachen Stickstoff-Strom ständig aus dem Reaktionsgemisch vertrieben. Nach dem Abkühlen wird das Gemisch 1 Stunde mit der Lösung von 5 g Natriumhydrogencarbonat in 100 ml Wasser gerührt, die organische Phase abgetrennt, über $Na_2SO_4$ getrocknet, mit Kohle entfärbt und im Vakuum eingedampft. Der verbleibende ölige Rückstand wird 3 Stunden bei 50°C und 0,1 mbar getrocknet, man erhält 20,3 g (63,5 % d.Th.) der Verbindung Nr. 3 als hellbraunes Harz.

IR-Spektrum (KBr): 3112, 3050, 2992, 2950, 2922, 1740, 1660, 1446, 1277, 1195, 1132, 1098, 955, 814, 782, 746, 600 $cm^{-1}$.

Entsprechend werden folgende Verbindungen hergestellt.

Beispiel 4 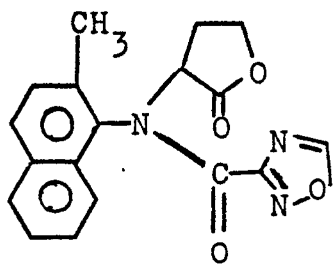 Schmp. 132-136°C

Beispiel 5 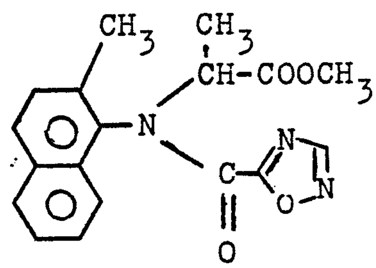 Harz

Beispiel 6 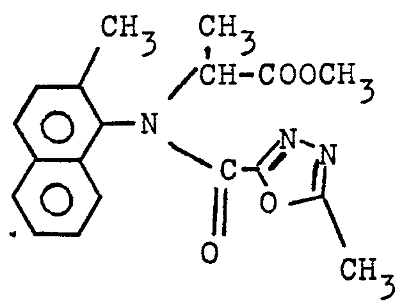 Harz

Beispiel 7 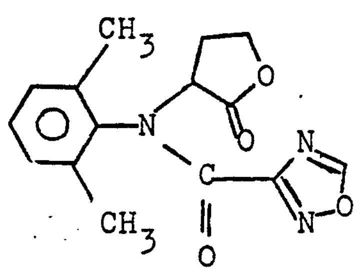 Schmp. 128-130°C

Beispiel 8 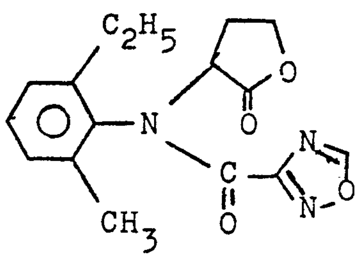 IR [KBr]: 3128, 2980, 2932, 1775, 1660, 1428, 1382, 1290, 1225, 1180, 1028, 790 $cm^{-1}$

Beispiel 9 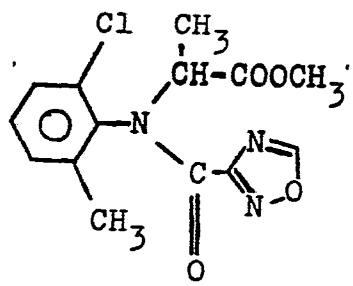 Harz

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Erysiphe graminis an Getreide, Uncinula necator an Reben, Podophaera leucotricha an Äpfeln, Sphaerotheca fuliginea an Rosen, Erysiphe cichoriacearum an Gurken. Die fungiziden Mittel enthalten 0,1 bis 95 % (Gewichtsprozent) Wirkstoff, vorzugsweise 0,5 bis 90 %. Die Aufwandmengen liegen ja nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Darüber hinaus sind viele der neuen Verbindungen systemisch wirksam, so daß über die Wurzelbehandlung auch ein Schutz oberirdischer Pflanzenteile möglich ist.

Die neuen Wirkstoffe können auch zusammen mit anderen Wirkstoffen, z.B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt.

Manganethylenbisdithiocarbamat
Mangan-Zinkethylenbisdithiocarbamat
Ammoniak-Komplex von Zink-(N,N-ethylen-bis-dithiocarbamat)
N-Trichlormethylthio-tetrahydrophthalimid
N-Trichlormethyl-phthalimid
5-Ethoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid
N-Cyclohexyl-N-methoxy-2,5-dimethyl-furan-3-carbonsäure-amid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Wirkstoffen kombiniert werden können, sind beispielsweise:

Dithiocarbamate und deren Derivate, wie
Ferridimethyldithiocarbamat
Zinkdimethyldithiocarbamat
Zinkethylenbisdithiocarbamat
Tetramethylthiuramdisulfide
Zink-(N,N-propylen-bis-dithiocarbamat)
Ammoniak-Komplex von Zink-(N,N'-propylen-bis-dithiocarbamat)
und
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5-b)-chinoxalin
1-(Butylcarbamoyl)-2-benzimidazol-carbaminsäuremethyl-ester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-oxid
2,3-Dihydro-5-carboxanilido-6-methyl-1,4-oxathiin
2-(Furyl-(2))-benzimidazol
Piperazin-1,4-diyl-bis-(1-(2,2,2-trichlor-ethyl)-formamid
2-(Thiazolyl-(4))-benzimidazol

5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glutarimid
Hexachlorbenzol
N-Dichlorfluormethylthio-N',N'-dimethyl-N-phenyl-schwefelsäureamid
2,5-Dimethyl-furan-3-carbonsäureanilid
2-Methyl-benzoesäure-anilid
2-Jod-benzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol.

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten, durch Versprühen, vernebeln, Verstäuben, Verstreuen, Beizen oder Gießen angewendet. Die Aufwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen

von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht:
Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes

Isooctylphenol-, Octylphenol-, Nonylphenol, Alkylphenolpolyglykolether, Tributylphenylpolyglykolether, Alkalarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Für die folgenden Versuche wurde als bekannter Vergleichswirkstoff die folgende Verbindung verwendet.

N-(1-Methoxycarbonylethyl)-N-(pyridin-3-carbonyl)-2-methyl-6-chloranilin A (bekannt aus DE-OS 25 13 732).

Versuch 1

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß beispielsweise die Verbindungen 3 und 4 bei der Anwendung als 0,025%ige Spritzbrühe eine bessere fungizide Wirkung haben (beispielsweise 97 %) als der bekannte Wirkstoff A (beispielsweise 70 %).

Versuch 2

Wirksamkeit gegen Phytophthora infestans an Tomaten

Blätter von Topfpflanzen der Sorte "Große Fleischtomate" werden mit wäßriger Spritzbrühe, die 80 % Wirkstoff und 20 % Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise die Wirkstoffe 3, 4 und 7 bei der Anwendung als 0,025%ige Spritzbrühe eine bessere fungizide Wirkung haben (beispielsweise 97 %) als der bekannte Wirkstoff A (beispielsweise 30 %).

Beispiele für Zubereitungen sind:

I. Man vermischt 90 Gew.-Teile der Verbindung 3 mit 10 Gew.-Teilen N-Methyl-alpha-pyrrolidon und erhält eine Lösung, die zur Anwendung in Form kleinster Tropfen geeignet ist.

II. 20 Gew.-Teile der Verbindung 3 werden in einer Mischung gelöst, die aus 80 Gew.-Teilen Xylol, 10 Gewichtsteilen des Anlagerungsproduktes von 8 bis 10 Mol Ethylenoxid an 1 Mol Ölsäure-N-monoethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung 4 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung 4 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung 3 werden mit 3 Gew.-Teilen des Natriumsalzes der Diisobutylnaphthalin-alpha--sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung 7 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung 3 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstiffs mit guter Haftfähigkeit.

VIII. 40 Gew.-Teile der Verbindung 4 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung 7 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Patentansprüche

1. Oxadiazolcarbonsäureamid der allgemeinen Formel

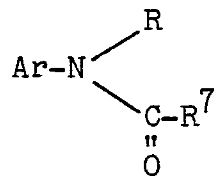 I,

worin

Ar 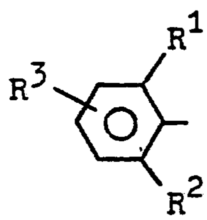 oder 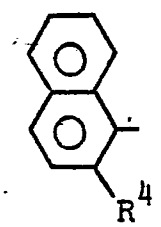 bedeutet, und

$R^1$ $C_1$-$C_3$-Alkyl, $C_1$-$C_3$-Alkoxy oder Halogen, $R^2$ $C_1$-$C_3$-Alkyl, $C_1$-$C_3$-Alkoxy, Halogen oder $NO_2$, $R^3$ Wasserstoff, $C_1$-$C_3$-Alkyl oder $C_1$-$C_3$-Alkoxy und $R^4$ Methyl bedeutet,

R 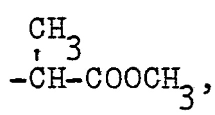, 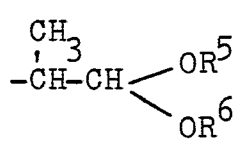 oder 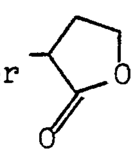 bedeutet, und

$R^5$ und $R^6$ Methyl oder Ethyl bedeuten oder $R^5$ und $R^6$ zusammen eine $C_2$-$C_3$-Alkylengruppe bedeuten, die zusammen mit dem Rest

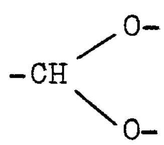 einen gegebenenfalls durch $C_1$-$C_3$-Alkyl

substituierten Dioxolan- oder 1,3-Dioxanring bilden und $R^7$ einen gegebenenfalls durch $C_1$-$C_3$-Alkyl substituierten 1,2,4-Oxadiazol-3-yl-, 1,2,4-Oxadiazol-5-yl- oder 1,3,4-Oxadiazol-2-yl-rest bedeutet.

2. Oxadiazolcarbonsäureamid gemäß Anspruch 1, <u>dadurch gekennzeichnet</u>, daß $R^1$ und $R^2$ gleich oder verschieden sind und Methyl, Ethyl, Methoxy, Ethoxy oder Chlor und $R^3$ Wasserstoff oder Methyl bedeutet.

3. Fungizide Mittel, enthaltend ein Oxadiazolcarbonsäureamid gemäß Anspruch 1 und einen inerten Trägerstoff.

4. Verfahren zur Bekämpfung von Pilzen, <u>dadurch gekennzeichnet</u>, daß man ein Oxadiazolcarbonsäureamid gemäß Anspruch 1 auf die Pilze oder auf durch Pilzbefall bedrohte Flächen, Pflanzen oder Saatgüter einwirken läßt.

5. Verfahren zur Herstellung von Verbindungen gemäß Anspruch 1, <u>dadurch gekennzeichnet</u>, daß man ein aromatisches Amin der Formel II

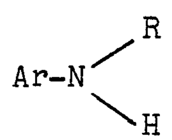

II,

worin
Ar und R die in Anspruch 1 angegebenen Bedeutungen haben,

a) mit einem Säurehalogenid der Formel III

$$R^7-\overset{\overset{O}{\|}}{C}-Hal \qquad III$$

oder

b) mit einem Säureanhydrid der Formel IV

$$(R^7\text{-}CO)_2O \qquad IV,$$

worin
$R^7$ die in Anspruch 1 angegebene Bedeutung hat und Hal Chlor oder Brom bedeutet, gegebenenfalls in Gegenwart eines Lösungsmittels, gegebenenfalls unter Zusatz einer anorganischen oder organischen Base und gegebenenfalls unter Zusatz eines Reaktionsbeschleunigers bei Temperaturen zwischen -10 und 100°C umsetzt.

6. Verfahren zur Herstellung von Verbindungen gemäß Anspruch 1, dadurch gekennzeichnet, daß man eine Verbindung der Formel 1, worin $R^5$ und $R^6$ Methyl oder Ethyl bedeuten, mit einem 1,2- bzw. 1,3-Diol unter Abspaltung von Methanol oder Ethanol, gegebenenfalls in Gegenwart eines Lösungsmittels und gegebenenfalls unter Zusatz eines sauren Katalysators zu einer Verbindung der Formel I umsetzt, in der $R^5$ und $R^6$ zusammen mit der Gruppe 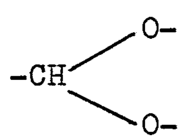 einen Dioxolan- bzw. 1,3-Dioxanring bilden.

Patentansprüche (für Österreich)

1. Fungizides Mittel enthaltend ein Oxadiazolcarbonsäureamid der allgemeinen Formel

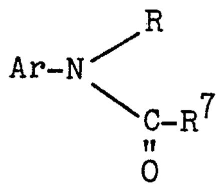 I,

worin

Ar 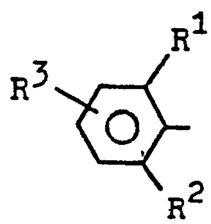 oder 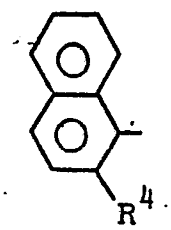 bedeutet, und

$R^1$ $C_1$-$C_3$-Alkyl, $C_1$-$C_3$-Alkoxy oder Halogen, $R^2$ $C_1$-$C_3$-Alkyl, $C_1$-$C_3$-Alkoxy, Halogen oder $NO_2$, $R^3$ Wasserstoff, $C_1$-$C_3$-Alkyl oder $C_1$-$C_3$-Alkoxy und $R^4$ Methyl bedeutet,

R 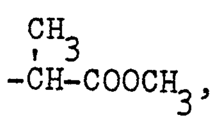, 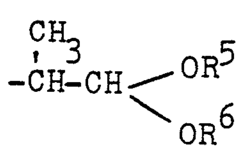 oder 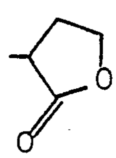 bedeutet, und

$R^5$ und $R^6$ Methyl oder Ethyl bedeuten oder $R^5$ und $R^6$ zusammen eine $C_2$-$C_3$-Alkylengruppe bedeuten, die zusammen mit dem Rest

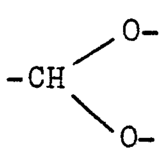 einen gegebenenfalls durch $C_1$-$C_3$-Alkyl

substituierten Dioxolan- oder 1,3-Dioxanring bilden und $R^7$ einen gegebenenfalls durch $C_1$-$C_3$-Alkyl substituierten 1,2,4-Oxadiazol-3-yl-, 1,2,4-Oxadiazol-5-yl- oder 1,3,4-Oxadiazol-2-yl-rest bedeutet.

2. Fungizides Mittel gemäß Anspruch 1, dadurch gekennzeichnet, daß $R^1$ und $R^2$ gleich oder verschieden sind und Methyl, Ethyl, Methoxy, Ethoxy oder Chlor und $R^3$ Wasserstoff oder Methyl bedeutet.

3. Fungizide Mittel, enthaltend ein Oxadiazolcarbonsäureamid gemäß Anspruch 1 und einen inerten Trägerstoff.

4. Verfahren zur Bekämpfung von Pilzen, dadurch gekennzeichnet, daß man ein Oxadiazolcarbonsäureamid gemäß Anspruch 1 auf die Pilze oder auf durch Pilzbefall bedrohte Flächen, Pflanzen oder Saatgüter einwirken läßt.

5. Verfahren zur Herstellung von Verbindungen gemäß Anspruch 1, dadurch gekennzeichnet, daß man ein aromatisches Amin der Formel II

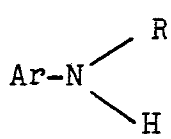

II,

worin
Ar und R die in Anspruch 1 angegebenen Bedeutungen haben,

a) mit einem Säurehalogenid der Formel III

$$R^7-\overset{O}{\overset{\|}{C}}-Hal \qquad III$$

oder

b) mit einem Säureanhydrid der Formel IV

$$(R^7\text{-CO})_2O \qquad IV,$$

worin $R^7$ die in Anspruch 1 angegebene Bedeutung hat und Hal Chlor oder Brom bedeutet, gegebenenfalls in Gegenwart eines Lösungsmittels, gegebenenfalls unter Zusatz einer anorganischen oder organischen Base und gegebenenfalls unter Zusatz eines Reaktionsbeschleunigers bei Temperaturen zwischen -10 und 100°C umsetzt.

6. Verfahren zur Herstellung von Verbindungen gemäß Anspruch 1, dadurch gekennzeichnet, daß man eine Verbindung der Formel 1, worin $R^5$ und $R^6$ Methyl oder Ethyl bedeuten, mit einem 1,2- bzw. 1,3-Diol unter Abspaltung von Methanol oder Ethanol, gegebenenfalls in Gegenwart eines Lösungsmittels und gegebenenfalls unter Zusatz eines sauren Katalysators zu einer Verbindung der Formel I umsetzt, in der $R^5$ und $R^6$ zusammen mit der Gruppe 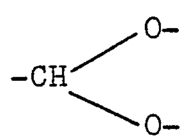 einen Dioxolan- bzw. 1,3-Dioxanring bilden.